# DEMANDE DE BREVET EUROPEEN

(11) **EP 1 430 872 A1**
(43) Date de publication de la demande: **23.06.2004**
(21) Numéro de dépôt: 03292687.5
(22) Date de dépôt: 28.10.2003
(51) Int. Cl.: A61K 7/075

(54) **Compositions cosmétiques contenant un tensioactif amphotère et un hydroxyacide et leurs utilisations**

(30) Priorité: 19.12.2002 FR 0216197
(71) Demandeur: L'OREAL, 75008 Paris (FR)
(72) Inventeur: Maubru, Mireille, 78400 Chatou (FR)
(74) Mandataire: Le Blainvaux Bellegarde, Françoise

(57) **Abrégé**

L'invention concerne de nouvelles compositions cosmétiques notamment détergentes comprenant dans un milieu cosmétiquement acceptable au moins un tensioactif amphotère choisi parmi les composés alcoyl amphohydroxyalkyl sulfonates et leurs sels, et au moins 1 % en poids d'au moins d'un hydroxyacide.

Cette association apporte des propriétés cosmétiques améliorées notamment le lissage, la légèreté et le démêlage des fibres kératiniques.

Ces compositions sont utilisées notamment pour le lavage et/ou le conditionnement des matières kératiniques telles que les cheveux ou la peau.

## Description

La présente invention concerne de nouvelles compositions cosmétiques, notamment détergentes comprenant dans un milieu cosmétiquement acceptable au moins un tensioactif amphotère choisi parmi les composés alcoylamphohydroxyalkylsulfonates et leurs sels et au moins 1% en poids d'au moins un hydroxyacide.

Pour le nettoyage et/ou le lavage des cheveux et/ou de la peau, l'utilisation de compositions détergentes (shampooing ou gel-douche) à base essentiellement d'agents tensioactifs classiques de type notamment anionique, non ionique et/ou amphotère, mais plus particulièrement de type anionique, est courante. Ces compositions sont appliquées sur cheveux ou peau mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur les matières kératiniques des dommages plus ou moins marqués liés en particulier à l'élimination progressive des lipides ou protéines contenues dans ou à la surface de ces dernières.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les tensioactifs amphotères alcoylamphohydroxyalkylsulfonates ont déjà été préconisés dans des compositions cosmétiques détergentes. Ils ont été décrits par exemple dans la demande de brevet WO99/36054.

Les compositions de lavage des cheveux utilisant ces tensioactifs seuls ne conduisent pas à de propriétés cosmétiques satisfaisantes.

Par ailleurs, il est déjà connu d'utiliser des hydroxyacides dans des composirtions cosmétiques notamment dans des shampooings dans le but d'améliorer les propriétés mécaniques, en particulier la résistance. Cependant, ces compositions de lavage des cheveux ne conduisent pas à de propriétés cosmétiques satisfaisantes.

L'invention a donc pour but de proposer des compositions cosmétiques détergentes présentant des propriétés cosmétiques améliorées, en particulier le démêlage, le lissage, la souplesse ou la malléabilité des cheveux.

Or, la demanderesse a maintenant trouvé que l'association d'hydroxyacide dans une quantité supérieure à 1% en poids et d'un tensioactif amphotère alcoylamphohydroxyalkylsulfonate permettait d'atteindre ces buts.

Les compositions conformes à l'invention confèrent aux matières kératiniques notamment les cheveux, un remarquable effet traitant qui se manifeste notamment par une facilité de démêlage, ainsi qu'un apport de légèreté, de lissage, de malléabilité et de souplesse sans aucune sensation de toucher chargé.

L'invention a ainsi pour objet une composition cosmétique comprenant, dans un milieu cosmétiquement acceptable, au moins un tensioactif amphotère alcoylamphohydroxyalkylsulfonate et ses sels et au moins 1% en poids d'au moins d'un hydroxyacide.

Un autre objet de l'invention concerne l'utilisation d'au moins un tensioactif amphotère tel que défini ci-dessus dans, ou pour la fabrication d'une composition cosmétique comprenant au moins 1% en poids d'au moins d'un hydroxyacide.

Un autre objet de l'invention concerne un procédé de traitement des matières kératiniques, telles que les cheveux, caractérisé en ce qu'il consiste à appliquer sur lesdites matières des compositions cosmétiques selon l'invention.

L'invention a encore pour objet l'utilisation d'une composition selon l'invention pour améliorer le démêlage ou le lissage des cheveux, ou pour apporter du volume, de la légèreté, de la souplesse ou de la malléabilité aux cheveux.

Selon la présente invention, par matières kératiniques, on comprend les cheveux, les cils, les sourcils, la peau, les ongles, les muqueuses ou le cuir chevelu et plus particulièrement les cheveux.

Les différents objets de l'invention vont maintenant être détaillés. L'ensemble des significations et définitions des composés utilisés dans la présente invention données ci-dessous sont valables pour l'ensemble des objets de l'invention.

Les tensioactifs amphotères alcoylamphohydroxyalkylsulfonates peuvent avoir la formule suivante (I) : 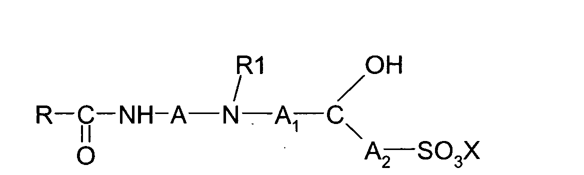 dans laquelle :
R désigne un radical hydrocarboné, saturé ou insaturé, linéaire ou ramifié comportant de 5 à 29 atomes de carbone.
R désigne de préférence un radical alkyle ou alkényle mono ou polyinsaturé comportant de 5 à 29 atomes de carbone et de préférence de 7 à 22 atomes de carbone et encore plus particulièrement de 9 à 17 atomes de carbone.
R1 désigne un radical hydroxyalkyle en C₁-C₄, de préférence hydroxyéthyle,
A, A1, A2 , identiques ou différents, désignent un radical alkylène divalent linéaire ou ramifié en C₁-C₁₀, de préférence en C₁-C₃
X désigne un atome d'hydrogène, un cation minéral ou organique tel que:
   celui d'un métal alcalin (par exemple Na⁺, K⁺) , celui d'un métal alcalinoterreux (Mg²⁺, Ca²⁺) , l'ion NH₄⁺, les ions ammoniums issus des aminoacides basiques ou des amino-alcools.

Des composés préférés selon la présente invention sont des composés de formule (II) dans laquelle R désigne plus particulièrement un radical alkyle saturé, linéaire ou ramifié comportant de 7 à 29 atomes de carbone, de préférence de 7 à 22 atomes de carbone.

Lorsque X désigne un ion ammonium issu d'alcanolamine, celle-ci peut être la monoéthanolamine, la diéthanolamine, la triéthanolamine, l'amino-3 propanediol-1,2. Lorsque X désigne un ion ammonium issu d'amine, cette amine peut être un aminoacide basique tel que la lysine, l'arginine, la sarcosine, l'ornithine ou la citrulline.

De préférence, A= A2 et désigne -CH₂CH₂-.

De préférence, A1 désigne -CH₂-

De préférence X désigne Na⁺

Parmi les tensioactifs de formule (I), on peut citer plus particulièrement les sels de Cocoyl amphohydroxypropyl sulfonate et notamment le sel de sodium tel que le produit proposé sous la dénomination MIRANOL CSE par la société RHODIA CHIMIE ou encore les sels de palmitoyl amphohydroxypropyl sulfonate.

Selon l'invention, le ou les tensioactifs amphotères choisis parmi les composés alcoylamphohydroxyalkylsulfonates et leurs sels peuvent représenter de 0,1 % à 30 % en poids, de préférence 1% à 20% en poids et plus particulièrement de 1,5 % à 15 % en poids par rapport au poids total de la composition finale.

Selon une caractéristique essentielle des compositions cosmétiques selon l'invention, ces dernières contiennent au moins 1% en poids d'un hydroxyacide ou de ses dérivés.

Par dérivés d'acide, on entend ses sels associés (sels avec une base organique ou un alcalin notamment) ou bien encore éventuellement son lactide correspondant (forme obtenu par autoestérification des molécules).

Cet hydroxyacide peut bien entendu consister en un acide mono ou poly carboxylique comportant une ou plusieurs fonctions hydroxy. L'hydroxyacide peut être un alpha ou un bétahydroxyacide. De préférence l'hydroxyacide est un alpha hydroxy acide. Les positions alpha et béta traduisent le fait qu'une au moins des fonctions hydroxy occupe une position alpha ou béta par rapport à au moins une des fonctions carboxy de l'acide, c'est à dire est rattachée respectivement soit au carbone porteur de la fonction carboxy soit au carbone adjacent de celui qui est porteur de la fonction carboxy. Cet acide peut se présenter dans la composition finale sous forme d'acide libre et/ou sous la forme de l'un de ses sels associés (sels avec une base organique ou un alcalin notamment), en particulier selon le pH final imposé à la composition, ou bien encore éventuellement sous la forme du lactide correspondant (forme obtenu par autoestérification des molécules). Les compositions conformes à l'invention peuvent bien entendu contenir un ou plusieurs hydroxyacides ou leurs dérivés.

A titre d'exemples de tels composés, on peut citer, entre autres, les acides salicylique, citrique, lactique, méthyllactique, phényllactique, malique, mandélique, glycolique, tartronique, tartrique, gluconique, benzylique et 2-hydroxycaprylique. D'autres composés de type hydroxyacides convenant à la présente invention sont ceux cités dans la demande de brevet EP-A- 0 413 528, dont l'enseignement est, à cet égard, totalement inclus dans la présente demande.
De préférence, on retiendra les acides qui sont cosmétiquement compatibles et acceptables avec les cheveux, la peau et/ou le cuir chevelu.

Selon un mode particulièrement préféré de réalisation de la présente invention, l'hydroxyacide mis en oeuvre est choisi parmi l'acide citrique, l'acide tartrique et l'acide lactique.

Selon une autre caractéristique importante des compositions cosmétiques selon l'invention, le ou les hydroxyacides sont présents dans ces dernières à raison d'au moins 1 % en poids, de préférence au moins 2 % en poids par rapport à l'ensemble de la composition. Des teneurs en hydroxyacide(s) variant de 2 à 10 % en poids, et plus particulièrement encore de 3 à 6 % en poids, conviennent généralement très bien. On notera que ces concentrations sont nettement supérieures à celles qui peuvent parfois se rencontrer dans des shampooings de l'art antérieur lorsque certains acides ont été employés uniquement à des fins d'ajustement de pH.

De préférence, le rapport en poids tensioactif(s) amphotère(s) sulfonate de l'invention / hydroxyacide de l'invention est compris entre 0,1 et 20 et plus particulièrement entre 0,5 et 15.

Les compositions de l'invention contiennent en outre avantageusement au moins un autre agent tensioactif qui est généralement présent en une quantité comprise entre 0,1% et 40% en poids environ, de préférence entre 3% et 30% et encore plus préférentiellement entre 5% et 20%, par rapport au poids total de la composition.

Cet agent tensioactif peut être choisi parmi les agents tensioactifs anioniques, amphotères, non-ioniques, ou leurs mélanges.

Les tensioactifs additionnels convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates, de cocoyl iséthionate et d'alkyléthersulfates et leurs mélanges.

On utilise de préférence comme agent tensioactif anionique les alkyl(C₁₂-C₁₄) sulfates de sodium, de magnésium ou d'ammonium, les alkyl (C₁₂-C₁₄)éthersulfates de sodium, de magnésium ou d'ammonium oxyéthylénés à 2,2 moles d'oxyde d'éthylène, le cocoyl iséthionate de sodium et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères additionnels, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - S03H
R₅ désigne un radical alkyle d'un acide carboxylique présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 7ème édition, 1997, sous les dénominations Disodium Cocoamphodiacetate, Disodium Lauroamphodiacetate, Disodium Capryloamphodiacetate, Disodium Caproamphodiacetate, Disodium Cocoamphodipropionate, Disodium Lauroamphodipropionate, Disodium Caproamphodipropionate, Disodium Capryloamphodipropionate, Lauroamphodipropionic acid, Cocoamphodipropionic acid.
A titre d'exemple on peut citer le disodium cocoamphodiacetate commercialisé sous la dénomination commerciale MIRANOL® C2M concentré par la société RHODIA CHIMIE.

Dans les compositions conformes à l'invention, on utilise de préférence des mélanges d'agents tensioactifs et en particulier des mélanges d'agents tensioactifs anioniques ou des mélanges d'agents tensioactifs anioniques et d'agents tensioactifs amphotères ou non ioniques.

Le(s) agent(s) tensioactif(s) anionique(s) sont généralement présents à raison de 1 à 30 % en poids, de préférence de 3 à 15 % en poids, par rapport au poids total de la composition.

Le(s) agent(s) tensioactif(s) amphotère(s) (différents des composés sulfonates) ou non ioniques sont généralement présents à raison de 0,5 à environ 15% en poids, de préférence de 1 à 5% en poids, par rapport au poids total de la composition.

La quantité et la qualité des tensioactifs sont celles suffisantes pour conférer à la composition finale un pouvoir moussant et/ou détergent satisfaisant.

Dans la composition selon la présente invention, la totalité des tensioactifs détergents représente généralement de 4 à 50% en poids et de préférence de 6 à 35% en poids et plus particulièrement de 8 à 25% en poids par rapport au poids total de la composition.

Selon un mode préféré de l'invention, les compositions selon l'invention comprennent en outre un ou plusieurs polymères cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société AMERCHOL, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société ONDEO, les polysaccharides cationiques non cellulosiques dont plus particulièrement les gommes de guar modifiées par du chlorure de 2,3-époxypropyl triméthylammonium commercialisées par exemple sous la dénomination « JAGUAR C13S » par la société RHODIA CHIMIE.
On peut également utiliser les polymères comprenant des motifs récurrents répondant à la formule : 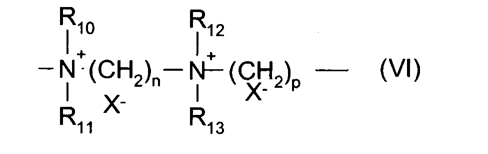 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Les compositions conformes à l'invention peuvent contenir en plus de l'association définie ci-dessus des agents régulateurs de viscosité tels que des électrolytes, ou des agents épaississants. On peut citer en particulier le chlorure de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants et/ou opacifiants bien connus dans l'état de la technique tels que par exemple les oxydes de titane enrobés ou non, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses ( par exemple en C8-C30) tels que par exemple le distéaryléther ou le 1-(hexadécyloxy)-2-octadécanol, les cyclodextrines, les alcools gras tels que les alcools cétylique, stéarylique et béhénylique.

Les compositions selon l'invention peuvent contenir également des synergistes de mousses tels que des 1,2-alcanediols en C₁₀-C₁₈ ou des alcanolamides gras dérivés de mono ou de diéthanolamine.

La composition de l'invention peut également contenir au moins un additif choisi parmi les parfums, les conservateurs, les filtres solaires, les tensioactifs cationiques, les polymères anioniques ou non ioniques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoique, les vitamines, les provitamines telles que le panthénol, les huiles végétales, les huiles minérales et les huiles de synthèse, les agents antipelliculaires et tout autre additif classiquement utilisé dans le domaine cosmétique qui n'affecte pas la stabilité et les propriétés des compositions selon l'invention.

Ces additifs éventuellement sont présents dans la composition selon l'invention dans des proportions pouvant aller de 0,001 à 50% en poids par rapport au poids total de la composition. La quantité précise de chaque additif est déterminée facilement par l'homme du métier selon sa nature et sa fonction.

Le milieu cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.
De préférence , la composition comprend de 50 à 95 % en poids d'eau par rapport au poids total de la composition.

Les compositions détergentes selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent être utilisées pour le lavage et le traitement des matières kératiniques telles que les cheveux, la peau, les cils, les sourcils, les ongles, les lèvres, le cuir chevelu et plus particulièrement les cheveux.

En particulier, les compositions détergentes selon l'invention sont des shampooings, des gels-douche et des bains moussants.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de produits démaquillants.

Les compositions selon l'invention peuvent se présenter sous forme de gel, de lait, de crème, d'émulsion, de lotion épaissie ou de mousse et être utilisées pour la peau, le cuir chevelu, les ongles, les cils, les lèvres et plus particulièrement les cheveux.

Ces compositions détergentes sont de préférence moussantes et le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1 m. La quantité de composition qui chute est de 200 ml. Ces 200 ml de composition tombent dans une éprouvette ayant un diamètre de 50 mm et contenant 50 ml de la composition à tester. La mesure est faite 5 minutes après l'arrêt de l'écoulement de la composition.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage en particulier avec de l'eau.

Ainsi, ce procédé selon l'invention permet le traitement, le soin, le lavage ou le démaquillage de la peau, des cheveux ou de toute autre matière kératinique.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits. Dans les exemples, MA signifie matière active.

### EXEMPLE 1 :

On a réalisé deux compositions de shampooings, l'une conforme à l'invention (composition A) et l'autre comparative (composition B) :

| Composition | Invention | Comparatif |
|---|---|---|
| Lauryléthersulfate de sodium (C12/C14 à 70/30) à 2.2 moles d'oxyde d'éthylène | 11.2 g M.A. | 11.2 g M.A. |
| Cocoyl amphohydroxypropyl sulfonate de sodium, en solution aqueuse à 32% de matière active, proposé sous la dénomination Miranol CSE par la société RHODIA | 3.0 g M.A. | - |
| Cocoamidoéthyl (N-hydroxyéthyl, N-carboxyméthyl) glycinate de sodium, vendu sous la dénomination Miranol C2M par la société RHODIA | - | 3.0 g M.A. |
| Hydroxyéthylcellulose réticulée par l'épichlorhydrine et quaternisée par la triméthylamine, vendue sous la dénomination JR 400 par la société AMERCHOL | 0.8 g | 0.8 g |
| Distéarate d'éthylène glycol | 2.0 g | 2.0 g |
| Acide citrique | 3.0 g | 3.0 g |
| Acide polyacrylique réticulé | 0.2 g | 0.2 g |
| Conservateurs | q.s. | q.s. |
| Parfum | q.s. | q.s. |
| Ammoniaque q.s. | pH 5.3 | pH 5.3 |
| Eau déminéralisée q.s.p. | 100.0 g | 100.0 g |

On effectue un shampooing en appliquant environ 6 g de la composition A sur une demi-tête de cheveux naturels préalablement mouillés. On fait mousser le shampooing puis on rince abondamment à l'eau. On sèche les cheveux avec un sèche-cheveux.
On procède selon le même mode opératoire que ci-dessus avec la composition comparative B sur l'autre demi-tête.
Les experts comparent les demi-têtes deux à deux.
Un panel d'experts a évalué l'aspect des cheveux séchés et a noté
- une plus grande facilité de démêlage
- un meilleur lissage (visuel et au toucher).

Les cheveux traités avec la composition A sont significativement plus lisses et se démêlent plus facilement, que ceux traités avec la composition B.

### EXEMPLES 2 à 4

On a préparé les compositions de shampooing suivantes :

| Composition | Exemple 2 | Exemple 3 | Exemple 4 |
|---|---|---|---|
| Lauryléthersulfate de sodium (C12/C14 à 70/30) à 2.2 moles d'oxyde d'éthylène | 11.2 g MA | 11.2 g MA | 11.2 g MA |
| Cocoyl amphohydroxypropyl sulfonate de sodium, en solution aqueuse à 32% de matière active, (Miranol CSE par la société RHODIA) | 3 g MA | 3 g MA | 3 g MA |
| Hydroxyéthylcellulose réticulée par l'épichlorhydrine et quaternisée par la triméthylamine (JR 400 par la société AMERCHOL) | 0.8 g | 0.8 g | 0.3 g |
| Distéarate d'éthylène glycol | 2 g | 2 g | 2 g |
| Acide citrique | 3 g | 3 g | 3 g |
| Acide polyacrylique réticulé | 0.2 g | 0.2 g | 0.2 g |
| Conservateurs | q.s. | q.s. | q.s. |
| Parfum | q.s. | q.s. | q.s. |
| Soude (NaOH) q.s. | - | - | pH 5.3 |
| Aminométhyl propanol q.s. | - | pH 5.3 | - |
| Ammoniaque q.s. | pH 5.3 | - | - |
| Eau déminéralisée q.s.p. | 100 g | 100 g | 100 g |

Les cheveux lavés avec ces compositions sont lisses, souples et malléables.

## Revendications

1. Composition cosmétique, **caractérisée par le fait qu'**elle comprend, dans un milieu cosmétiquement acceptable au moins un tensioactif amphotère choisi parmi les composés alcoylamphohydroxyalkylsulfonates et leurs sels et au moins 1% en poids d'au moins un hydroxyacide.

2. Composition selon la revendication 1, **caractérisée par le fait que** ledit tensioactif amphotère est choisi parmi les composés alcoylamphohydroxyalkylsulfonates ayant la formule suivante (I) : 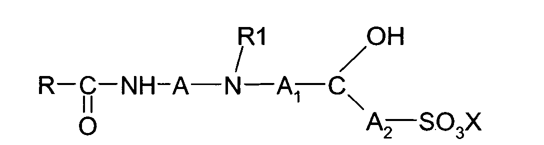 dans laquelle :
R désigne un radical hydrocarboné, saturé ou insaturé, linéaire ou ramifié comportant de 5 à 29 atomes de carbone.
R1 désigne un radical hydroxyalkyle en C₁-C₄, de préférence hydroxyéthyle,
A, A1, A2 , identiques ou différents, désignent un radical alkylène divalent linéaire ou ramifié en C₁-C₁₀, de préférence en C₁-C₃
X désigne un atome d'hydrogène, un cation minéral ou organique.

3. Composition selon la revendication 2, **caractérisée par le fait que** R désigne de préférence un radical alkyle ou alkényle mono ou polyinsaturé comportant de 5 à 29 atomes de carbone et de préférence de 7 à 22 atomes de carbone et encore plus particulièrement de 9 à 17 atomes de carbone.

4. Composition selon l'une quelconque des revendications 2 ou 3, **caractérisée par le fait que** A et A2 désignent -CH₂CH₂-.

5. Composition selon l'une quelconque des revendications 2 à 4, **caractérisée par le fait que** A1 désigne -CH₂-.

6. Composition selon l'une quelconque des revendications 2 à 5, **caractérisée par le fait que** la cation minéral ou organique est choisi parmi celui d'un métal alcalin ( par exemple Na⁺, K⁺) , celui d'un métal alcalinoterreux (Mg²⁺, Ca²⁺) , l'ion NH₄⁺, les ions ammoniums issus des aminoacides basiques ou des amino-alcools.

7. Composition selon l'une quelconque des revendications 1 à 6, **caractérisée par le fait que** ledit tensioactif amphotère est choisi parmi les sels de Cocoyl amphohydroxypropyl sulfonate et les sels de palmitoyl amphohydroxypropyl sulfonate.

8. Composition selon l'une quelconque des revendications 1 à 7, **caractérisée en ce que** le ou les tensioactifs amphotères alcoylamphoallkylsulfonates et leurs sels sont présents à une concentration comprise entre 0,1 et 30 % en poids, de préférence entre 1% et 20% en poids par rapport au poids total de la composition.

9. Composition selon l'une quelconque des revendications précédentes, **caractérisée par le fait que** l'hydroxyacide est présent sous forme d'acide libre et/ou sous la forme de l'un de ses sels associés et/ou sous la forme du lactide correspondant.

10. Composition selon l'une quelconque des revendications précédentes, **caractérisée par le fait que** l'hydroxyacide est un alpha hydroxyacide.

11. Composition selon l'une quelconque des revendications 1 à 9, **caractérisée par le fait que** l'hydroxyacide est un béta hydroxyacide.

12. Composition selon la revendications 10, **caractérisée par le fait que** l'alphahydroxyacide est choisi parmi les acides citrique, lactique, méthyllactique, phényllactique, malique, mandélique, glycolique, tartronique, tartrique, gluconique, benzylique et 2-hydroxycaprylique.

13. Composition selon la revendication 11, **caractérisée par le fait que** le bêta hydroxyacide est l'acide salicylique.

14. Composition selon l'une quelconque des revendications 1 à 13, **caractérisée par le fait que** ledit hydroxy acide est présent dans des proportions allant de 2 à 10% en poids par rapport au poids total de la composition, de préférence 3 à 6% en poids.

15. Composition selon l'une quelconque des revendications 1 à 14, **caractérisée par le fait qu'**elle comprend en outre au moins un agent tensioactif additionnel choisi parmi les tensioactifs anioniques, cationiques, non ioniques, amphotères et leurs mélanges.

16. Compositions selon la revendication 15, **caractérisées par le fait que** le ou les agents tensioactifs additionnels sont présents à une concentration comprise entre 0,1% et 40% en poids, de préférence entre 3% et 30% en poids, et encore plus préférentiellement entre 5% et 20% en poids, par rapport au poids total de la composition.

17. Composition selon l'une quelconque des revendications précédentes, **caractérisée par le fait qu'**elle contient en outre une ou plusieurs silicones.

18. Composition selon l'une quelconque des revendications précédentes, **caractérisée par le fait qu'**elle contient en outre une ou plusieurs polymères cationiques.

19. Composition selon la revendication 18, **caractérisée par le fait que** lesdits polymères cationiques sont choisis parmi :
- les homopolymères de sel de diallyldiméthylammonium et les copolymères de sel de diallyldiméthylammonium et d'acrylamide,
- les dérivés d'éther de cellulose quaternaires
- les polysaccharides cationiques non cellulosiques
- les polymères comprenant des motifs récurrents répondant à la formule : 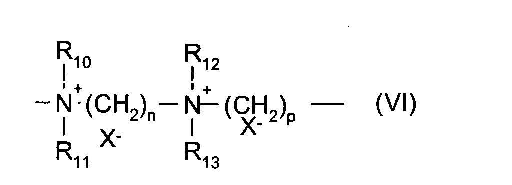
dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.

20. Composition selon l'une quelconque des revendications 18 à 19, **caractérisée en ce que** le polymère cationique est présent à une concentration comprise entre 0,001 % et 10 % en poids par rapport au poids total de la composition, de préférence entre 0,005 % et 5 % en poids.

21. Composition selon l'une quelconque des revendications précédentes, **caractérisée par le fait qu'**elle contient en outre au moins un agent nacrant et/ou opacifiant.

22. Composition selon la revendication 21, **caractérisée par le fait que** d'agents nacrants et/ou opacifiants sont choisis parmi les oxydes de titane enrobés ou non, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses , le distéaryléther ou le 1-(hexadécyloxy)-2-octadécanol, les cyclodextrines, les alcools gras tels que les alcools cétylique, stéarylique et béhénylique.

23. Composition selon l'une quelconque des revendications 1 à 22, **caractérisée par le fait qu'**elle comprend en outre au moins un additif choisi parmi les épaississants, les parfums, les conservateurs, les filtres solaires, les polymères anioniques ou non ioniques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoique, les vitamines, les provitamines telles que le panthénol, les huiles végétales, les huiles minérales, les huiles de synthèse, les agents antipelliculaires.

24. Composition selon l'une quelconque des revendications 1 à 23, **caractérisées par le fait qu'**elle se présente sous forme de shampooing, de composition lavantes pour la peau, d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

25. Utilisation d'une composition telle que définie dans l'une quelconque des revendications précédentes pour le lavage des matières kératiniques en particulier les cheveux.

26. Utilisation d'une composition telle que définie dans l'une quelconque des revendications 1 à 24 pour améliorer le démêlage ou le lissage des cheveux , ou pour apporter du volume, de la légèreté, de la douceur, de la souplesse ou de la malléabilité aux cheveux.

27. Utilisation d'un tensioactif amphotère alcoylampho hydroxyalkyl sulfonate ou ses sels dans une composition cosmétique contenant au moins 1% en poids d'un hydroxyacide pour améliorer le démêlage ou le lissage des cheveux, ou pour apporter du volume, de la légèreté, de la douceur, de la souplesse ou de la malléabilité aux cheveux.

28. Procédé de traitement des matières kératiniques, telles que les cheveux, **caractérisé en ce qu'**il consiste à appliquer sur lesdites matières une composition cosmétique selon l'une quelconque des revendications 1 à 24, puis à effectuer éventuellement un rinçage.
